(19) Europäisches Patentamt / European Patent Office / Office européen des brevets

(11) **EP 4 208 085 B1**

(12) **EUROPEAN PATENT SPECIFICATION**

(45) Date of publication and mention of the grant of the patent:
**09.07.2025 Bulletin 2025/28**

(21) Application number: **21765586.9**

(22) Date of filing: **11.08.2021**

(51) International Patent Classification (IPC):
**A61B 5/0295** (2006.01)   **A61B 5/1455** (2006.01)
**A61B 5/029** (2006.01)

(52) Cooperative Patent Classification (CPC):
**A61B 5/14551; A61B 5/02007; A61B 5/0295;**
**A61B 5/4818; A61B 5/6826; A61B 5/7239;**
**A61B 5/7246; A61B 5/7282;** A61B 5/029

(86) International application number:
**PCT/EP2021/072349**

(87) International publication number:
**WO 2022/048871 (10.03.2022 Gazette 2022/10)**

(54) **METHOD AND APPARATUS FOR DETECTING SLEEP-DISTURBING EVENTS FROM A SIGNAL INDICATIVE OF A PERIPHERAL ARTERIAL TONE OF AN INDIVIDUAL**

VERFAHREN UND VORRICHTUNG ZUR ERKENNUNG VON SCHLAFSTÖRUNGSEREIGNISSEN AUS EINEM SIGNAL, DAS EINEN PERIPHEREN ARTERIELLEN TON EINER PERSON ANZEIGT

PROCÉDÉ ET APPAREIL POUR DÉTECTER DES ÉVÉNEMENTS PERTURBANT LE SOMMEIL À PARTIR D'UN SIGNAL INDICATIF D'UN TONUS ARTÉRIEL PÉRIPHÉRIQUE D'UN INDIVIDU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.09.2020   US 202063074960 P**

(43) Date of publication of application:
**12.07.2023   Bulletin 2023/28**

(73) Proprietor: **Ectosense NV**
**3110 Rotselaar (BE)**

(72) Inventors:
• **VITS, Steven**
**1060 Sint-Gillis (BE)**
• **MASSIE, Frederik**
**3000 Leuven (BE)**

(74) Representative: **IP HILLS NV**
**Bellevue 5/501**
**9050 Gent-Ledeberg (BE)**

(56) References cited:
**EP-A1- 1 259 157**   **US-A1- 2016 120 465**
**US-A1- 2019 099 125**   **US-A1- 2020 015 737**

## Description

### Technical Field

The present disclosure relates to a method and an apparatus for detecting sleep-disturbing events, and more specifically, for detecting sleep-disturbing events from a signal indicative of a peripheral arterial tone, PAT, of an individual.

### Background

Plethysmography, be it pneumatic-based or optics-based, is a measurement technique commonly used to monitor changes in blood volume in the macrovascular and the microvascular system of an individual and, more specifically, to monitor changes in the blood volume comprised in the arteries or arterioles. The changes in the arterial blood volume are further affected by the contractions of the muscular walls of the arteries or arterioles. Monitoring the changes in the arterial blood volume with plethysmography therefore empirically provides information on relative changes in muscle tension or 'tone' of the smooth muscle tissue of the arterioles, also referred to as peripheral arterial tone, PAT.

Optical plethysmography or photoplethysmography measures the arterial blood volume changes by shining light from one or more light sources, such as LEDs, onto an investigated volume and by detecting collected light corresponding to the light being reflected or transmitted in the investigated volume on a sensor, wherein the sensor may, for example, comprise or correspond to a photodetector, such as for example a photodiode. The light sources and the sensor form the so-called plethysmography probe and can for example be arranged on opposite sides of the investigated volume, e.g., a finger, a nostril, an ear, a forehead, the inside of a mouth, a toe, a wrist, an ankle, etc., of an individual, hereby allowing the measurement of a transmission mode photo-plethysmogram, PPG, or at the same side of the investigated volume, hereby allowing the measurement of a reflectance mode PPG. Irrespective of the employed measurement technique, both the transmission mode PPG and the reflectance mode PPG capture the cyclic fluctuations of arterial blood volume in the investigated volume and, thus, changes in the peripheral arterial tone of the individual.

Because arterial blood flow to the investigated volume can be modulated by multiple other physiological phenomena such as breathing and heart rate, plethysmography can also be used for monitoring respiratory and circulatory conditions, hypovolemia, and even for detecting sleep-disturbing events leading to or caused by sleep disorders. Sleep disorder diagnosis is a medical field wherein a patient's sleep is monitored during a certain time, e.g., one or more nights. Based on the monitoring, different sleep-related or, better, sleep-disturbing events may be identified such as apneic events, snoring, limb movements, or others. For example, the reuptake of breathing at the end of a sleep apnea event usually coincides with a norepinephrine release. Norepinephrine is released in the bloodstream and binds to adrenergic receptors in the arterioles in the investigated volume, which triggers an increase in muscle tension of the arterioles resulting in a reduction of the arteriole diameter and a reduction of arterial blood volume in the investigated volume. Monitoring peripheral arterial tone, PAT, through plethysmography, for example, can therefore provide valuable information on the occurrence of these sleep-disturbing events such as, for example, sleep apnea. This observation is widely reported in the last century and explained in detail in the scientific publication entitled "Effect of pain on autonomic nervous system indices derived from photoplethysmography in healthy volunteers" by Hamunen et al. published in the British Journal of Anesthesia, Volume 108, Issue 5, P838-844, on May 01, 2012, where the authors describe that the Pulse PlethysmoGraphic Amplitude, or PPGA, of the photoplethysmograph is due to pulsatile changes in tissue volume, mainly arterial blood and that during sympathetic activation, or vasoconstriction, the PPGA decreases.

However, due to the effect of gravity, when some part of the body is situated below heart level, a hydrostatic pressure gradient is automatically generated. Hydrostatic pressure gradients affect the venous system, an innately low-pressure system, causing venous distention also known as venous blood pooling. Venous pooling can invoke a reflex constrictory response in the arteries supplying blood to the veins, thus imposing an additional physiological change that can obscure the desired arterial vasomotor response which is investigated by the plethysmography. This reflex is commonly referred to as venoarteriolar reflex or VAR and is described in detail in PART A of the whitepaper "An Illustrated Atlas of PAT Signals in Sleep Medicine" by Bar et al. To solve this problem, US7374540B2 proposes a plethysmography probe capable of completely covering the surface of the distal end of the finger and providing a uniform pressure field that extends all the way to the terminal end of the finger. US7374540B2 proposes a probe with an internal membrane and an external membrane with the inner membrane that applies a predetermined static pressure to the body part and the outer membrane that assures the predetermined static pressure applied by the inner membrane is substantially unaffected by volume changes of the body part. The external counter-pressure exerted by the outer membrane, thus, is claimed to reduce venous blood pooling and distension within the measurement site and is claimed to reduce distal venous pooling. This is claimed to reduce the likelihood of the induction of venoarteriolar reflex vasoconstriction, which can otherwise lead to the development of an indeterminate degree of vasoconstriction. The prior art, therefore, describes a complex and costly probe for providing a uniform pressure probe to reduce issues related to an indeterminate degree of vasoconstriction.

## Summary

It is an object of embodiments of the present disclosure to provide a solution capable of detecting sleep-disturbing events leading to or caused by sleep disorders overcoming the shortcomings on the conventional solutions, and, more specifically, to provide a solution capable of detecting sleep-disturbing events including sleep disorders in an accurate and robust way based on physiological information acquired by means of conventional, off-the-shelf medical equipment. It is a further object of embodiments of the present disclosure to provide a solution capable of detecting sleep-disturbing events including sleep disorders in an accurate and robust way based on a signal indicative of the peripheral tone of an individual acquired by conventional, off-the-shelf medical equipment such as plethysmography probes not providing uniform pressure.

The scope of protection sought for various embodiments of the invention is set out by the independent claims.

These objects are achieved, according to a first example aspect of the present disclosure, by a computer-implemented method for detecting sleep-disturbing events as defined by claim 1. In particular, the computer-implemented method is capable of detecting sleep-disturbing events from a signal indicative of a peripheral arterial tone, PAT, of an individual affected by a venoarteriolar reflex, VAR. The signal may be obtained by conventional, off-the-shelf medical equipment, as long as the obtained signal provides information indicative of the PAT and the effects on the signal caused by the venoarteriolar reflex, VAR. Examples of such medical equipment include standard probes employing pneumatic-plethysmography or photo-plethysmography, PPG, probes capable of obtaining a PAT signal affected by VAR. The method, thus, comprises processing that signal obtained from the PPG probe to derive therefrom sleep-disturbing events. More specifically, the method comprises determining one or more vasoconstriction events from changes in the signal. The method further comprises deriving a reference amplitude value and a baseline amplitude value for the determined vasoconstriction event. The reference amplitude value and the baseline amplitude value are different from each other. The reference and baseline amplitude values correspond to the amplitude values of characteristics points in the signal for the vasoconstriction event which allow characterization of the vasoconstriction event. The obtained reference and baseline amplitude values are then related to each other to obtain a measure indicative of the magnitude of the vasoconstriction event, i.e., a magnitude measure characterizing the intensity or significance of the vasoconstriction event and, therefore, the intensity or significance of the observed physiological event. Once the magnitude of the detected physiological event is obtained, the method proceeds to detect, therefrom, a sleep-disturbing event. Relating the reference amplitude value to a baseline amplitude value allows for adequately reducing the effects of more slowly varying, i.e., steady-state changes, observed in the signal. Steady-state changes are effects observed in the signal that are varying at a slower rate than the changes of the signal resulting from the vasoconstriction events. As detailed above, such steady-state changes may, for example, be caused by a venoarteriolar reflex. In other words, the reference and baseline amplitude values are related to each other such that the steady-changes in the signal are taken into account and, thus, the venoarteriolar reflex, when determining the magnitude of a vasoconstriction event. Doing so assures a correct assessment of the magnitude of the vasoconstriction events and, in turn, correct detection and characterization of the sleep-disturbing events, therefrom. As a result, false positive and false negative detection of sleep-disturbing events is substantially eliminated.

The reference and baseline amplitude values for the vasoconstriction event are preferably derived from a baseline-invariant version of the signal. This baseline-invariant signal may be calculated specifically for the vasoconstriction event or for the complete signal. That is, a partial baseline-invariant signal for the vasoconstriction event or a baseline-invariant signal for the complete signal can be calculated. From the baseline-invariant signal, the reference amplitude value and the baseline value for the vasoconstriction event are then selected. As detailed above, the reference and baseline amplitude values correspond to the amplitude values of characteristics points in the signal for the vasoconstriction event which allow characterization of the vasoconstriction event. The selection of the reference and baseline amplitude values is thus preferably done in a way that allows the calculation of the magnitude measure for the vasoconstriction event.

The baseline-invariant signal is preferably calculated by dividing the obtained signal with a baseline. The dividing can be performed with respect to the portion of the signal corresponding to the vasoconstriction event or the complete signal. In the first case, the baseline-invariant signal for the vasoconstriction event is calculated by dividing the portion of the signal corresponding to the vasoconstriction event with the baseline for the event. The baseline is a baseline level which may be a baseline value or a signal portion for the corresponding vasoconstriction event. In the second case, the baseline-invariant signal is obtained by dividing the complete signal with a baseline for the complete signal. Herein, the baseline is a baseline signal. Doing so results in removing the venoarteriolar reflex effect on the arterial tone for at least the vasoconstriction event, thus assuring correct assessment of the magnitude of the vasoconstriction events and therefore a correct detection and characterization of the sleep-disturbing events. The reference amplitude value and baseline amplitude value are then be derived from the baseline-invariant signal. For example, the maximum amplitude value observed in the portion of the baseline-invariant signal corresponding to the vasoconstriction event can be selected as the reference amplitude value and the mean amplitude value observed in the portion of the baseline-invariant signal corresponding to the vasoconstriction event can be selected as the baseline amplitude value.

The thus selected reference amplitude value and baseline amplitude value are related to each other to obtain the magnitude measure for the vasoconstriction event. Preferably, the reference amplitude value is related to the baseline amplitude value by calculating, from the reference amplitude value and the baseline amplitude value, an absolute magnitude for the vasoconstriction event. As the reference and the baseline amplitude values are selected from the baseline-invariant version of the signal, the magnitude for the vasoconstriction event can be derived, for example, as the difference between them. The thus calculated magnitude measure is an absolute value that allows quantifying the intensity of the vasoconstriction event on an absolute scale.

The baseline for the vasoconstriction event is preferably derived by calculating an envelope of the signal. Calculating the envelope of the signal allows extracting the venoarteriolar reflex observed in the signal. In other words, the calculated signal's envelope characterizes the venoarteriolar reflex throughout the complete signal. A peak envelope, a troughs envelope, a peak-to-troughs average envelope, a percentile value-based envelope, or a smoothed version of the signal can, for example, be calculated and used as the envelope of the signal. Alternatively, instead of calculating the envelope of the complete signal, an amplitude value for the vasoconstriction event may be calculated which can be used as a baseline for the vasoconstriction event. For example, a peak amplitude, a through amplitude, a peak-to-trough average amplitude, or a percentile value for the vasoconstriction event can be calculated and used as the baseline for the vasoconstriction event.

Alternatively, the reference amplitude value and the baseline amplitude value for the vasoconstriction event can be directly derived from the obtained signal. This can be done by, for example, calculating a peak amplitude, a through amplitude, a peak-to-trough average amplitude, or a percentile value for the vasoconstriction event directly from the obtained signal. These amplitude values may serve as a baseline amplitude value or a reference amplitude value for the vasoconstriction event as long as the baseline and reference amplitude values are selected to differ from one another. For example, the peak amplitude for the vasoconstriction event can be selected as the reference amplitude value and any of other amplitude values characterizing the vasoconstriction event, i.e., through amplitude, peak-to-though average amplitude, or average amplitude, as the baseline amplitude value.

The thus selected reference amplitude value and baseline amplitude values are then related to each other to obtain the magnitude measure for the vasoconstriction event. Preferably, the reference amplitude value is related to the baseline amplitude value by calculating, from the reference amplitude value and the baseline amplitude value for the vasoconstriction event, a relative magnitude for the vasoconstriction event. The relative magnitude for the vasoconstriction event can be derived, for example, as their relative difference or relative change or any other substantially relative magnitude measure. The, thus calculated magnitude measure is a relative value that allows quantifying the intensity of the vasoconstriction event on a relative scale.

The detecting of the sleep-disturbing event preferably comprises identifying a vasoconstriction event characterized by a magnitude measure above a pre-determined value. In other words, vasoconstriction events with a certain magnitude measure are considered as sleep disturbing events. The pre-determined value can be determined based on, for example, measurements obtained during clinical trials. It is preferred that the step of detecting sleep-disturbing events includes using a classifier trained or developed for said detecting. Any conventional classifier, such as a neural network, a decision tree, or a support vector machine, may be trained.

Preferably, the step of identifying further takes into account at least one of a duration of the vasoconstriction event, a duration of an amplitude drop period and/or a duration amplitude rise period of the vasoconstriction event, steepness of the amplitude drop period, and/or steepness of the amplitude rise period of the vasoconstriction event. The duration of the vasoconstriction event, the duration of the amplitude drop period, and/or the duration of the amplitude rise period are additional measures that can be used to improve the quantification of the intensity of the vasoconstriction event. Similarly, the steepness of the amplitude drop and/or the steepness of the amplitude rise are other additional measures that can be also used to improve the quantification of the intensity of the vasoconstriction event. Taking any of these additional measures in combination with the magnitude measure allows quantification of the intensity of the vasoconstriction event in terms of various signal properties and, thus, improving the detection of the sleep-disturbing events and as well as their better characterization.

The determining of a vasoconstriction event preferably comprises identifying a portion of the signal characterized by an amplitude drop followed by an amplitude increase. In other words, to identify vasoconstriction events, the method searches for changes in the signal characterized by an amplitude drop followed by an amplitude increase. The determining can be performed by any suitable for the purpose signal processing technique.

Preferably, the determining further takes into account at least one of a duration of the amplitude drop period and the amplitude rise period of the signal portion, a duration of an amplitude drop period and/or a duration amplitude rise period of the signal portion, steepness of the amplitude drop period, and/or steepness of the amplitude rise period of the signal portion. Taking into account the duration of the amplitude drop and/or rise period, and the steepness of these respective periods allows quantification of the amplitude in terms of various signal properties and, thus, improving the determination of vasoconstriction events from the obtained signal.

The signal indicative of the peripheral arterial tone of the individual affected by a venoarteriolar reflex is preferably obtained by means of plethysmography. Plethysmography allows to obtain a signal, i.e., a plethysmography signal or plethysmogram, which is indicative of the changes in blood volume such as pulsatile blood volume changes, at a selected anatomical location of the patient, for example, a finger, a nostril, an ear, a forehead, the inside of a mouth, a toe, a wrist, an ankle, etc. A plethysmography signal can be obtained by so-called plethysmography probes which can employ pneumatic-based or optics-based plethysmography. As this plethysmography signal empirically provides information on relative changes in the muscle tension or 'tone' of the muscle tissue of the arterioles as well as information on the venoarteriolar reflex, the plethysmography signal allows deriving, therefrom, the signal indicative of changes in peripheral arterial tone which is affected by a venoarteriolar reflex.

Preferably, the signal indicative of the peripheral arterial tone is derived from an optical plethysmography signal measured at an investigated volume of the individual and light intensities acquired by means of optical plethysmography at two or more points in time along the optical plethysmography signal. Therefrom, changes in arterial blood volume in the investigated volume between the two or more points in time are derived by determining a logarithm or a function approximation thereof of a function of the light intensities, thereby assessing the peripheral arterial tone, PAT, of the individual. The logarithm or the function approximation of the function of the light intensities is herein referred to as an evaluation function. Preferably, this evaluation function corresponds to the logarithm of the ratio of the light intensities and the evaluation function depends on one or more of the following: an optical path length; a function of the oxygen saturation estimate or SpO2; and the changes in arterial blood volume in the investigated volume. Preferably, at least one of the points in time corresponds to the diastole in a cardiac cycle of the individual and/or wherein at least one of the points in time corresponds to the systole in a cardiac cycle of the individual.

According to a second example aspect, an apparatus configured to detect sleep-disturbing events from a signal indicative of a peripheral arterial tone of an individual affected by a venoarteriolar reflex is disclosed characterized by the features of claim 13. The apparatus comprises at least one processor and at least one memory including computer program code, where the at least one memory and computer program code are configured to, with the at least one processor, cause the apparatus to perform the steps of the first example aspect. Such an apparatus can thus provide one or more of the advantages mentioned with respect to the first example aspect.

According to a third example aspect, a system is disclosed comprising the apparatus according to the second example aspect. Preferably, the system further comprises a plethysmography probe. Preferably, the plethysmography probe is an optical plethysmography probe which comprises a light source configured to emit light and a sensor configured to collect by optical plethysmography propagated light corresponding to the light being transmitted or reflected when propagating in the investigated volume of the individual at the two or more points in time. The sensor is further configured to determine the light intensities of the propagated light at the two or more points in time. Optionally, the probe is further provided with an accelerometer to detect the position of the investigated volume. Optionally, the system can further comprise a wireless transmitter comprising a wireless communication interface, wherein the wireless transmitter is configured to transmit the determined peripheral arterial tone wirelessly for further processing by the apparatus. The wireless communication interface is preferably a low-power communication interface, e.g., a Bluetooth Low Energy, BLE, wireless interface. Such a system can thus provide one or more of the advantages with respect to the first example aspect.

According to a fourth example aspect, a use of a logarithm or a function approximation thereof for assessing a signal indicative of the peripheral arterial tone, PAT, of an individual monitored by optical plethysmography, is provided, wherein assessing the signal indicative of the peripheral arterial tone comprises: obtaining an optical plethysmography signal measured at an investigated volume of the individual and light intensities acquired by optical plethysmography at two or more points in time along the optical plethysmography signal; and determining changes in arterial blood volume in the investigated volume between the two or more points in time by determining a logarithm or a function approximation thereof of a function of the light intensities, thereby assessing PAT of the individual. The use of the logarithm or the function approximation allows obtaining a signal indicative of the PAT of an individual based on light intensities measured by means of optical plethysmography.

According to a fifth example aspect, a computer program product is disclosed comprising computer-executable instructions for causing a computer to perform the method according to the first example aspect.

According to a sixth example aspect, a computer readable storage medium is disclosed comprising computer-executable instructions for causing a computer to perform the method according to the first example aspect.

Such a computer program product and a computer readable storage medium can provide one or more of the advantages with respect to the first example aspect.

## Brief Description of the Drawings

Some example embodiments will now be described with reference to the accompanying drawings.

FIG.1A shows a simplified block schematic of a system for detecting sleep-disturbing events according to an example embodiment of the present disclosure;

FIG.1B shows steps for detecting sleep-disturbing events according to an example embodiment of the present disclosure;

FIG.2 shows an example of vasoconstriction events derived from a signal indicative of a peripheral arterial tone according to an example embodiment of the present disclosure;

FIG.3 shows a comparative example of a signal indicative of a peripheral arterial tone pre-processed according to an example embodiment of the present disclosure and the signal in its original, unprocessed form;

FIG.4A shows a comparative example of a signal indicative of a peripheral arterial tone pre-processed according to an example embodiment of the present disclosure and the signal in its original, unprocessed form;

FIG.4B shows various amplitude values for vasoconstriction events serving as a baseline amplitude value according to an example embodiment of the present disclosure;

FIG.5 shows various characteristic points in the pre-processed signal according to an example embodiment of the present disclosure;

FIG.6 shows an example of how the end-stage of respiratory events coincides with characteristic phenomena observed in various physiological signals; and

FIG.7 shows an example embodiment of a suitable computing system for performing one or several steps in embodiments of the invention.

## Detailed Description of Embodiment(s)

The present disclosure generally relates, amongst others, to a method and an apparatus for assessing a signal indicative of the peripheral arterial tone, PAT, of an individual for detecting sleep-related or, better, sleep-disturbing events. More particularly, the assessment of the signal is performed in such a way as to ensure substantial invariance to any steady-state changes observed in the signal, which may for example be caused by physiological phenomena such as a venoarteriolar reflex, thus ensuring accurate and robust detection of the sleep-disturbing events.

In the context of the present disclosure, an investigated volume of an individual is for example a volume defined in an investigated tissue of the individual which is monitored by pneumatic-based or optics-based plethysmography. Optical plethysmography is a measurement technique in which emitted light is collected on a sensor by optical plethysmography. In other words, an investigated volume of an individual is for example a volume defined in an investigated tissue of the individual for which an optical plethysmography signal is acquired. For example, the investigated volume is a peripheral tissue volume of the individual. For example, the investigated volume is a volume defined in finger, a tip of a finger, a distal end of a digit of the individual, a nostril, an ear, a forehead, the inside of a mouth, a toe, a tip of a toe, a wrist, an ankle of the individual. In the context of the present disclosure, the investigated volume of an individual comprises the skin of the individual comprised in the investigated volume and further comprises the blood volume present in the investigated volume.

In the context of the present disclosure, arterial blood volume is to be understood as the arterial blood volume in the investigated volume. In the context of the present disclosure, peripheral arterial tone or PAT is understood as arterial tone changes in investigated arterial beds in the investigated volume of an individual. In other words, determining pulsatile volume changes in the vascular beds of the investigated volume of the individual allows determining or assessing information indicative for muscle tension or 'tone' of the smooth muscle tissue of the arterioles in the investigated volume and therefore allows determining or assessing peripheral arterial tone which is modulated by the sympathetic nervous system. Determining peripheral arterial tone is non-invasive and can for example be used to detect heart diseases, erectile dysfunction, sleep apnea, obstructive sleep apnea, cardiovascular conditions, etc.

In the context of the present disclosure, an optical plethysmography signal is a signal measured by optical plethysmography. For example, the optical plethysmography signal is an optical plethysmogram. For example, the optical plethysmography signal is a PPG signal. The optical plethysmography signal is for example measured at the tip of a finger of the individual by an optical plethysmography setup comprising at least one light source and a sensor. In the context of the present disclosure, a light intensity corresponds to the intensity of light collected on the sensor of the optical plethysmography setup, wherein the light collected on the sensor corresponds to light generated by one or more light sources being transmitted through or reflected in the investigated volume of the individual.

In the context of the present disclosure, the oxygen saturation estimate or SpO2 or hemoglobin composition corresponds to a fraction of oxygenated hemoglobin related to a total amount of hemoglobin in the arterial blood volume in the investigated volume. For example, the oxygen saturation estimate or SpO2 or hemoglobin composition corresponds to a ratio of the concentration of oxygenated hemoglobin on the sum of the concentrations of oxygenated and deoxygenated hemoglobin in the arterial blood volume being monitored in the investigated volume. Alternatively, the oxygen saturation estimate or SpO2 or hemoglobin composition corresponds to a ratio of the volume fraction of oxygenated hemoglobin on the sum of the volume fractions of oxygenated and deoxygenated hemoglobin in the arterial blood volume being monitored in the investigated volume.

In the context of the present disclosure, deoxygenated hemoglobin is defined as the form of hemoglobin without the bound oxygen, and without any other bound molecule such as for example carbon monoxide, carbon dioxide, or iron. In the context of the present disclosure, oxygenated hemoglobin is defined as the form of hemoglobin with the bound oxygen. In the context of the present disclosure, light emitted by the light sources of an optical plethysmography setup comprises photons that reach the sensor through a probabilistic path of one or multiple scattering events. This optical path is not straight and is often assumed to follow a curved spatial probability distribution. The investigated volume along this curved optical path forms the volume which is sampled or investigated by optical plethysmography.

In the context of the present disclosure, one or more changes in arterial blood volume in the investigated volume between the two or more points in time are evaluated, thus assessing PAT of the individual. In the context of the present disclosure, a change in arterial blood volume in the investigated volume between two points in time corresponds to a relative change between the volume of arterial blood present in the investigated volume at a first point in time and the volume of arterial blood present in the investigated volume at a second point in time different from the first point in time.

In the context of the present disclosure, a chromophore is a molecular unit that absorbs or scatters light in the investigated volume. For example, in the context of the present disclosure, examples of chromophores are melanin molecules, oxygenated hemoglobin, deoxygenated hemoglobin, etc. In the investigated volume, the decay in light intensity of the incident light emitted by a light source of an optical plethysmography setup follows the Beer-Lambert law which can be formulated as in equation (1):

$$I = I_0 e^{-d \sum_i \varepsilon_i V_i - G(\lambda)} \qquad (1)$$

wherein:

- $I_0$ corresponds to the intensity of incident light emitted by a light source of an optical plethysmography setup;

- $\varepsilon_i$ comprises an absorption coefficient and/or a scattering coefficient and/or an extinction coefficient of chromophore i;

- $V_i$ corresponds to either a volume fraction or a concentration of chromophore i in the investigated volume;

- $d$ corresponds to the optical path length, wherein the optical path length corresponds to the path length a photon travels along before reaching the sensor of an optical plethysmography setup, wherein the optical path length is a function of, among others, the wavelength of the incident light and of chromophore composition in the investigated volume, and wherein the optical path length is dependent on the distance between the light source emitting the photon and the sensor. From the publication of Chatterjee et al. entitled "Monte Carlo Analysis of Optical Interactions in Reflectance and Transmittance Finger Photoplethysmography", published in Sensors (Basel) 19(4):789 on February 15 2019, doi:10.3390/s19040789, for a distance between the light source emitting the photons and the sensor of a few millimetres, such as for example 3 mm or less than 3mm, it can be approximated that the optical path length can be assumed constant;

- G corresponds to a scattering dependent light intensity loss parameter and which depends on the wavelength $\lambda$ of the incident light emitted by the light source.

Considering the following parameters:

- $V_{i,d}$ corresponding to either a volume fraction or a concentration of chromophore i in the investigated volume at a first point in time along the optical plethysmography signal;

- $V_{i,s}$ corresponding to either a volume fraction or a concentration of chromophore i in the investigated volume at a second point in time along the optical plethysmography signal;

- $I_h$ corresponding to the light intensity measured by the sensor of the optical plethysmography setup at the first point in time; and

- $I_l$ corresponding to the light intensity measured by the sensor of the optical plethysmography setup at the second point in time;

the Beer-Lambert law as formulated in Equation (1) can be evaluated at the first point in time and at the second point in time. When taking a ratio of both expressions, Equation (2) is obtained:

$$\frac{I_l}{I_h} = e^{-d \sum_i \varepsilon_i (V_{i,s} - V_{i,d})} \qquad (2)$$

Then, Equation (3) is obtained by taking the natural logarithm of both sides of equation (2), as follows:

$$ln\left(\frac{I_l}{I_h}\right) = -d \sum_i \varepsilon_i \left(V_{i,s} - V_{i,d}\right) \qquad (3)$$

If a logarithm with another base b that Euler's number e is used, Equation (3) then becomes:

$$\log_b\left(\frac{I_l}{I_h}\right) = \frac{-d \sum_i \varepsilon_i (V_{i,s} - V_{i,d})}{\ln(b)} \qquad (3')$$

It can be seen that Equation (3') is equal to Equation (3) up to the constant $\frac{1}{\ln(b)}$.

Equation (3) can be simplified to Equation (4) when the difference $V_{i,s}$ - $V_{i,d}$ is being renamed to $\Delta V_i$, thereby obtaining:

$$ln\left(\frac{I_l}{I_h}\right) = -d \sum_i \varepsilon_i \Delta V_i \qquad (4)$$

From Equation (4), it can be seen that the logarithm of the fraction of light intensities at the first point in time and at the second point in time is linearly related to the difference in either volume fraction or concentration of chromophore i between the first point in time and the second point in time.

Some chromophores remain attached to the epidermis of the individual between the two points in time along the optical plethysmography signal. For example, melanin molecules remain fixed to the investigated volume between the two points in time along the optical plethysmography signal. The difference in either volume fraction or concentration of such chromophores, such as for example melanin molecules, between the two points in time is therefore null. Hence, the contribution to the righthand side of Equation (4) of such chromophores is also null.

The main chromophores of which either the volume fraction or the concentration fluctuates between the two points in time along the optical plethysmography signal are the oxygenated and deoxygenated hemoglobin in the arterial blood volume. The two predominant forms of hemoglobin in the context of the present disclosure, i.e. oxygenated hemoglobin and deoxygenated hemoglobin, demonstrate absorption and scattering coefficients which are significantly different for most wavelengths of light.

The effect of all other chromophores, wherein all other chromophores are not oxygenated hemoglobin nor deoxygenated hemoglobin, of which either the volume fraction or the concentration fluctuates between the two points in time along the optical plethysmography signal can be written as the product of their combined extinction coefficient, $\varepsilon_{other}$, and one minus the sum of either the volume fractions or the concentrations of oxygenated hemoglobin and deoxygenated hemoglobin, wherein the sum of either all volume fractions or all concentrations equals 1.

Taking the above considerations into account, Equation (4) can then be rewritten as follows into Equation (5):

$$ln\left(\frac{I_l}{I_h}\right) = -d\big(\varepsilon_{HbO_2}\Delta V_{HbO_2} + \varepsilon_{Hb}\Delta V_{Hb} +$$
$$\varepsilon_{other}\left(1 - (V_{Hb,s} + V_{HbO_2,s}) - (1 - (V_{Hb,d} + V_{HbO_2,d}))\right)\big)$$

$$ln\left(\frac{I_l}{I_h}\right) = -d\left(\varepsilon_{HbO_2}\Delta V_{HbO_2} + \varepsilon_{Hb}\Delta V_{Hb} + \varepsilon_{other}\left(-(\Delta V_{Hb} + \Delta V_{HbO_2})\right)\right)$$

$$ln\left(\frac{I_l}{I_h}\right) = -d\left(\varepsilon_{HbO_2}\Delta V_{HbO_2} + \varepsilon_{Hb}\Delta V_{Hb} - \varepsilon_{other}(\Delta V_{Hb} + \Delta V_{HbO_2})\right)$$

$$ln\left(\frac{I_l}{I_h}\right) = -d\left((\varepsilon_{HbO_2} - \varepsilon_{other})\Delta V_{HbO_2} + (\varepsilon_{Hb} - \varepsilon_{other})\Delta V_{Hb}\right) \quad (5)$$

It is known that the oxygen saturation estimate can be defined according to Equation (6):

$$\frac{V_{HbO_2}}{V_{HbO_2} + V_{Hb}} = \text{SpO}_2 \qquad (6)$$

wherein:

- $V_{HbO_2}$ corresponds to either the volume fraction or the concentration of oxygenated hemoglobin comprised within the arterial blood in the investigated volume;
- $V_{Hb}$ corresponds to either the volume fraction or the concentration of deoxygenated hemoglobin comprised within the arterial blood in the investigated volume.

Additionally, $V_{blood}$ is defined as the total volume fraction or the total concentration of oxygenated and deoxygenated hemoglobin comprised within the arterial blood in the investigated volume and is defined as follows in Equation (7):

$$V_{HbO_2} + V_{Hb} = V_{blood} \qquad (7)$$

It is assumed that under normal circumstances, the sum of the volume fractions or the concentrations of oxygenated and deoxygenated hemoglobin within the arterial blood volume or the sum of the concentrations of oxygenated and deoxygenated hemoglobin within the arterial blood volume remains approximately constant throughout the measurement of the optical plethysmography signal. Indeed, only the ratio of oxygenated hemoglobin and deoxygenated hemoglobin, i.e., only the oxygen saturation estimate, might significantly change during the monitoring of the individual by optical plethysmography, for example during sleep apnea.
From Equation (6) and (7), the following can be obtained:

$$\text{SpO}_2 V_{blood} = V_{HbO_2}$$

$$(1 - \text{SpO}_2)V_{blood} = V_{Hb} \qquad (8)$$

By substituting expression (8) into Equation (5), the following can be obtained:

$$ln\left(\frac{I_l}{I_h}\right) = -d\left((\varepsilon_{HbO_2} - \varepsilon_{other})\text{SpO}_2 V_{blood} + (\varepsilon_{Hb} - \varepsilon_{other})(1 - \text{SpO}_2)V_{blood}\right) \qquad (9)$$

Two predetermined calibration coefficients $Q_1$ and $Q_2$, which are two constants, can be defined as follows:

$$Q_1 = \varepsilon_{HbO_2} - \varepsilon_{Hb}; \ Q_2 = \varepsilon_{Hb} - \varepsilon_{other}$$

After rewriting Equation (9) in view of the two predetermined calibration coefficients $Q_1$ and $Q_2$, Equation (10) can be obtained, wherein the left-hand side of Equation (10) corresponds to an evaluation function, and wherein $Q_1\text{SpO}_2 + Q_2$ corresponds to a compensation function which is a function of the oxygen saturation estimate:

$$ln\left(\frac{I_l}{I_h}\right) = -d\Delta V_{blood}(Q_1\text{SpO}_2 + Q_2) \quad\quad (10)$$

Equation (10) highlights a term on the left-hand side, referred to as the evaluation function, which shows a linear relationship with:

- the optical path length $d$;

- one or more changes in arterial blood volume $\Delta V_{blood}$, which is the parameter of interest; and

- a factor $(Q_1\text{SpO}_2 + Q_2)$, which linearly depends on the oxygen saturation estimate.

Under the assumption of a constant volume fraction or concentration of the sum of oxygenated and deoxygenated hemoglobin in the arterial blood, $\Delta V_{bood}$ is a linear proxy for arterial blood volume fluctuations within the investigated volume, and hence corresponds to a measurement of peripheral arterial tone.

For a constant value of the oxygen saturation estimate, i.e., $\text{SpO}_2 = const,$ a change in arterial blood volume in the investigated volume between two points in time is thus evaluated when determining the logarithm of the ratio of the light intensities collected on the sensor when measuring with optical plethysmography at said two points in time. Mathematically, this can be represented as

$$PAT_{channel} = ln\left(\frac{I_l}{I_h}\right) \sim d\Delta V_{blood}, \ if \ \text{SpO}_2 = const \quad\quad (11)$$

As only a linear relationship between a measurable parameter and $\Delta V_{blood}$ is relevant to investigate relative changes in $\Delta V_{blood}$ rather than the exact $\Delta V_{bood}$ value, determination of $\Delta V_{bood}$ up to a constant factor is sufficient, as is performed by determining the evaluation function.

If the $\text{SpO}_2$ is not constant, the evaluation function will also change with changes in $\text{SpO}_2$. However, compensation methods can be used to compensate for the effect of changes in $\text{SpO}_2$. For example, one can determine a compensation function $f_{\lambda_1}$, in which case Equation (11) can be re-written as:

$$PAT_{channel} = \frac{ln\left(\frac{I_l}{I_h}\right)}{f_{\lambda_1}(\text{SpO}_2)} \sim \Delta V_{blood} \quad\quad (12)$$

As a result, the $PAT_{channel}$ is a signal indicative of the peripheral arterial tone of the investigated volume of the individual. The $PAT_{channel}$ signal is thus a signal which can be obtained from the light intensities measured with optical plethysmography and the optical plethysmography signal by determining a logarithm or a function approximation thereof of a function of the light intensities which may be optionally divided by a function which depends on the SpO2, where the function of the light intensities corresponds to a ratio of the light intensities.

According to Equation (3), the evaluation function corresponds to the natural logarithm of a function of the light intensities. Alternatively, starting from Equation (2), any other evaluation function defined as a function of the light intensities could be used, for example, a linear approximation of the logarithm of a function of the light intensities, or for example a Taylor series approximation of a function of the light intensities, or for example a linear approximation of other base logarithms of a function of the light intensities. Alternatively, the evaluation function corresponds approximately to the ratio of the pulsatile waveform or AC component of the optical plethysmography signal and of the slowly varying baseline or DC component of the optical plethysmography signal, resulting in equation (13):

$$ln\left(\frac{I_l}{I_h}\right) = ln\left(\frac{I_l}{I_l} + \frac{I_h - I_l}{I_l}\right) = ln\left(1 + \frac{I_h - I_l}{I_l}\right) \cong \frac{I_h - I_l}{I_l} \equiv \frac{AC_{ppg}}{DC_{ppg}} \quad\quad (13)$$

The evaluation function thus corresponds to a logarithm of a ratio of the light intensities; and the evaluation function depends on one or more of the following:

- an optical path length;

- a function of the oxygen saturation estimate or SpO2;

- the changes in arterial blood volume in the investigated volume.

Further, at least one of the points in time corresponds to the diastole in a cardiac cycle of the individual and/or wherein at least one of the points in time corresponds to the systole in a cardiac cycle of the individual.

During systole, the volume of arterial blood in the investigated volume of the individual is maximum, resulting in the largest absorption and scattering of light of any point in time within a cardiac cycle, i.e., the period between two heart beats, since hemoglobin is one of the main absorbers and scatters of photons in the investigated volume, hence resulting in the lowest measured light intensity on the sensor of the optical plethysmography setup. Conversely, during diastole, the volume of arterial blood in the investigated volume of the individual is minimum, resulting in the lowest absorption and scattering of light of any point in time within a cardiac cycle and hence highest measured light intensity on the sensor of the optical plethysmography setup. At least one first point in time corresponds for example to the diastole in a first cardiac cycle and/or at least one second point in time corresponds for example to the systole in a second cardiac cycle different from the first cardiac cycle. Alternatively, at least one first point in time corresponds for example to the systole in a first cardiac cycle and/or at least one second point in time corresponds for example to the diastole in a second cardiac cycle different from the first cardiac cycle. Alternatively, at least one first point in time corresponds for example to the systole or to the diastole in a cardiac cycle and at least one second point in time corresponds to any point in time within the same cardiac cycle or within a different cardiac cycle.

As mentioned above the light intensities can be obtained by means of optical plethysmography by:

- emitting light at a wavelength by means of a light source; and

- collecting, by optical plethysmography and by a sensor, propagated light corresponding to light at the wavelength being transmitted or reflected when propagating in the investigated volume of the individual at the two or more points in time; and

- determining the light intensities of the propagated light on the sensor at the two or more points in time.

Optical plethysmography technology uses a simple and non-invasive setup probe or biosensor. The optical plethysmography biosensor non-invasively measures pulsatile volume changes in the investigated volume, and thereby assesses PAT, by collecting the optical plethysmography signal. The light source is for example a LED or any other suitable light source which can be miniaturized to fit in the optical plethysmography biosensor. The wavelength is for example comprised in the red spectrum. Alternatively, the wavelength is comprised in the infra-red spectrum. A physical distance between the light sources and the sensor is for example a few millimeters, such as for example less than 3mm.

The assessment of the signal indicative of the peripheral arterial tone, PAT, of an individual for detecting sleep-disturbing events will be now described in detail with reference to figures.

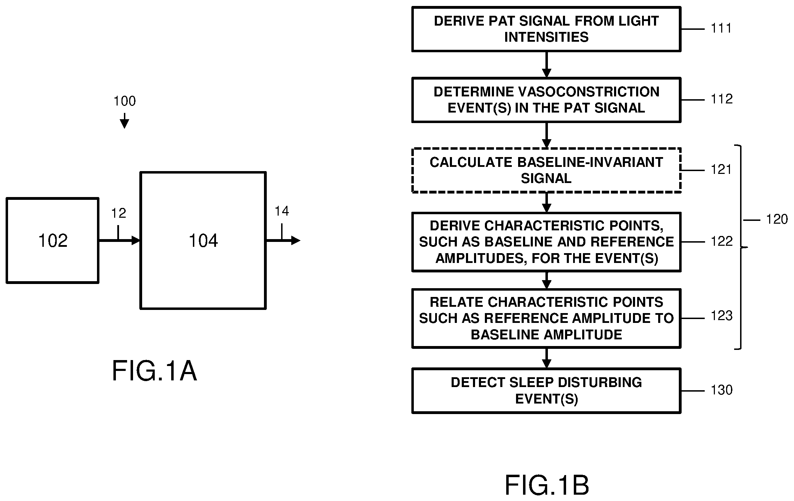

FIG.1A shows a simplified block scheme of a system according to an example embodiment of the present disclosure. The system 100 comprises a device 102 configured to measure fluctuations in arterial blood volume of an investigated volume of an individual by means of optical plethysmography and an apparatus 104 configured to obtain the light intensities measured by the device 102 and to process the obtained light intensities to detect, therefrom, sleep-disturbing events 14. The device 102 is for example placed on the finger of an individual. The device 102 thus measures the light intensity of the light propagated through the finger of the individual over time. As detailed above and illustrated in Equation (10) for example, the measured light intensities reflect the fluctuations in the arterial blood volume of the individual. Further, as detailed above and illustrated in Equations (11) and (12), the measured light intensities empirically provide information on the peripheral arterial tone of the individual. The device 102 thus outputs light intensities 12 measured over time reflecting the peripheral arterial tone of the individual. The measured light intensities are fed to the apparatus 104. The apparatus 104 comprises at least one processor and at least one memory configured to store algorithms to operate the apparatus stored in the form of software or program instructions in the at least one memory. In addition to storing the software, the at least one memory may further store any data generated by the apparatus and any other data required for its proper operation. This data may however be stored in another memory external to the apparatus. The at least one processor can execute the program instructions stored in the at least one memory and thus controls the operation of the apparatus. Otherwise said, in an embodiment, the apparatus 104 comprises a computing system that comprises hardware and software components for processing the obtained measured light intensities 12 and for determining therefrom sleep-disturbing events 14.

FIG.1B shows various steps performed by the apparatus 104 to detect sleep-disturbing events 14. In the first step, i.e., step 111, the peripheral arterial tone signal, i.e., the $PAT_{channel}$ signal, is derived from the obtained light intensities and the measured optical plethysmography signal as detailed above and illustrated in Equations (11) and (12). Summarized, the apparatus 104 obtains the light intensities 12 acquired over time by the device 102 by means of optical plethysmography, for example, at two or more points in time. From the obtained light intensities 12 the apparatus 104 then determines the changes in arterial blood volume in the investigated volume between the two or more points in time. This is done by determining a logarithm or a function approximation thereof of a function of the light intensities which may be optionally divided by a function that depends on the SpO2. The result is the $PAT_{channel}$ signal of the individual as illustrated in Equations (11) and (12).

In the next step, i.e., step 112, vasoconstriction events are determined from the $PAT_{channel}$ signal. As detailed above, vasoconstriction events are characterized by fluctuation in the $PAT_{channel}$ signal's amplitude. More specifically, a vasoconstriction event in the $PAT_{channel}$ signal is characterized with an amplitude drop followed by an amplitude increase. Vasoconstriction events in the $PAT_{channel}$ signal may be determined by means of any suitable for the purpose signal processing algorithms. Summarized, in this step, the $PAT_{channel}$ signal is segmented into event segments characterized by a drop, followed by an increase in the signal's amplitude. Each of these event segments reflects a vasoconstriction, i.e., constriction of the arteries in the investigated volume. In other words, the event segments correspond to the vasoconstriction events observed in the $PAT_{channel}$ signal. These vasoconstriction events may for example be related to sleep-disturbing events such as a respiratory event such as apnea or hypopnea, respiratory-related-arousal event or RERA event, periodic or non-periodic limb movement, bruxism event, or snore.

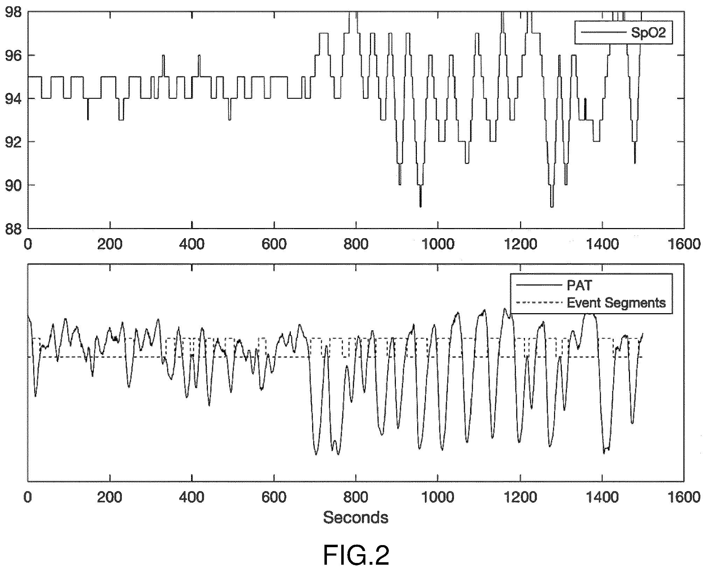

FIG.2 shows an example of the signal indicative of the peripheral arterial tone, PAT, of the individual with highlighted episodes of sudden arterial tone changes characterized by amplitude decreases followed by amplitude increases. As it can be seen, each of the detected event segments is characterized with an amplitude drop followed by an amplitude increase. The figure further shows that the fluctuations in the peripheral arterial tone of the respective event segments somewhat correlate to the fluctuations in oxygen saturation estimate and the SpO2 of the individual.

In the next step, i.e., step 120, the apparatus proceeds to characterize the respective event segments, i.e., the vasoconstriction events, in terms of characterizing features. The characterizing features comprise features such as for example: a magnitude of the event segment, i.e., the difference between the minimum and maximum amplitude values of the event segment; a duration of the event segment; a duration of the amplitude drop period and/or a duration of the amplitude rise period of the event segment; a steepness of the amplitude drop period and/or a steepness of the amplitude rise period of the event segment; the full width at half maximum of the event segment; and so on. The characterizing features of the respective event segments, however, need to be calculated so that the effect of the VAR episode on the amplitude in the event segments is substantially reduced.

To do so, the apparatus calculates in step 121 a baseline-invariant version of the $PAT_{channel}$ signal, i.e., a baseline-invariant signal. The baseline-invariant signal can be derived by relating the amplitude of the $PAT_{channel}$ signal to a baseline level, for example, by dividing the $PAT_{channel}$ signal by:

- a baseline signal corresponding to the upper amplitude of the $PAT_{channel}$ signal, i.e., its peak envelope or peak baseline;
- a baseline signal corresponding to the lower amplitude of the $PAT_{channel}$ signal, i.e., its troughs envelope or valley baseline;
- a baseline signal corresponding to the average of the upper and lower amplitude of the PAT channel, i.e., its peak-to-troughs average envelope;
- a baseline signal corresponding to any percentile amplitude value of the PAT channel, i.e., a percentile envelope;
- a smoothed version of any of the above, =
- a smoothed version of the $PAT_{channel}$ signal, e.g., by calculating a moving or sliding average version of the $PAT_{channel}$ signal with a window size longer than the duration of vasoconstriction events and a window sliding step corresponding to one or more samples of the $PAT_{channel}$ signal.

In case the baseline signal is any one of the smoothed signals mentioned above, it is important to ensure that the time-variation, for example, characterized by a time constant, of the smoothed signal is slow enough such that the resulting smoothed signal does not follow the shape of the event segment to such extent that the event segment's or $PAT_{channel}$ signal division by this smoothed signal results in a baseline-invariant signal with significant loss of information pertaining to the morphology of the original event segment. In other words, the fluctuations in the derived baseline-invariant signal caused by the peripheral arterial tone should be substantially preserved while the fluctuations caused by the VAR episode should be substantially reduced.

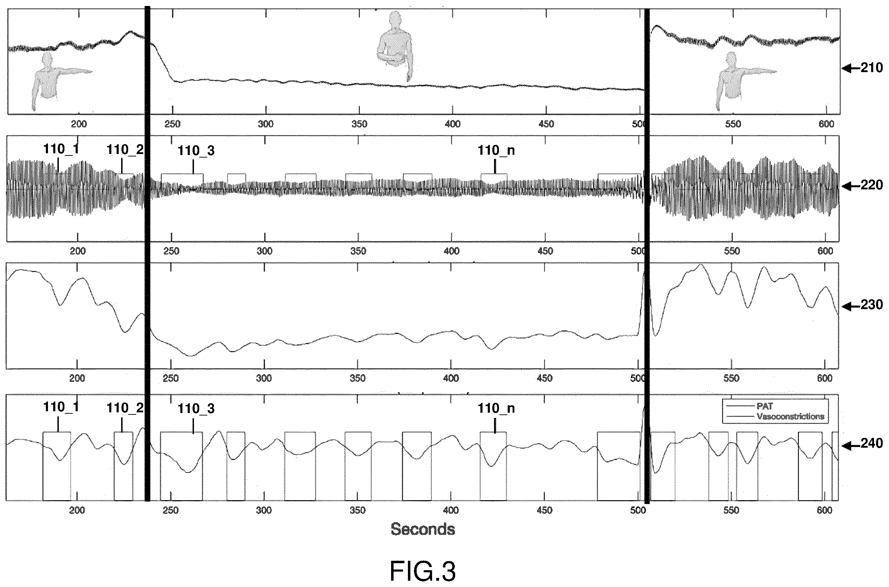

FIG.3 shows an example of the signal indicative of the peripheral arterial tone, PAT, of the individual affected by a VAR episode caused by venous blood pooling triggered by the lowering of the arm of the finger at which the optical plethysmography, PPG, signal is measured below the heart level, and highlights how the PAT channel amplitude is depressed during the VAR. More specifically, the top two plots show how the amplitude of the PPG signal 210 and the amplitude of its filtered and normalized version 220 become depressed during the VAR episode. The third plot shows how the amplitude of the PAT channel 230 derived as illustrated in Equations (11) or (12) drops during the VAR episode. The fourth plot shows how dividing each sample of the PAT channel by a baseline value results in a baseline-invariant version of the PAT channel 240 for which the effect of the VAR amplitude modulation is substantially eliminated. From the figure, it can be seen that the amplitude variations caused by the peripheral arterial tone in the event segments 110_1 to 110_3 and 110_n in the original PAT channel 220 and the baseline-invariant version of the PAT channel 240 are preserved while the amplitude modulation caused by the VAR episode is substantially eliminated in the baseline-invariant version of the PAT channel 240.

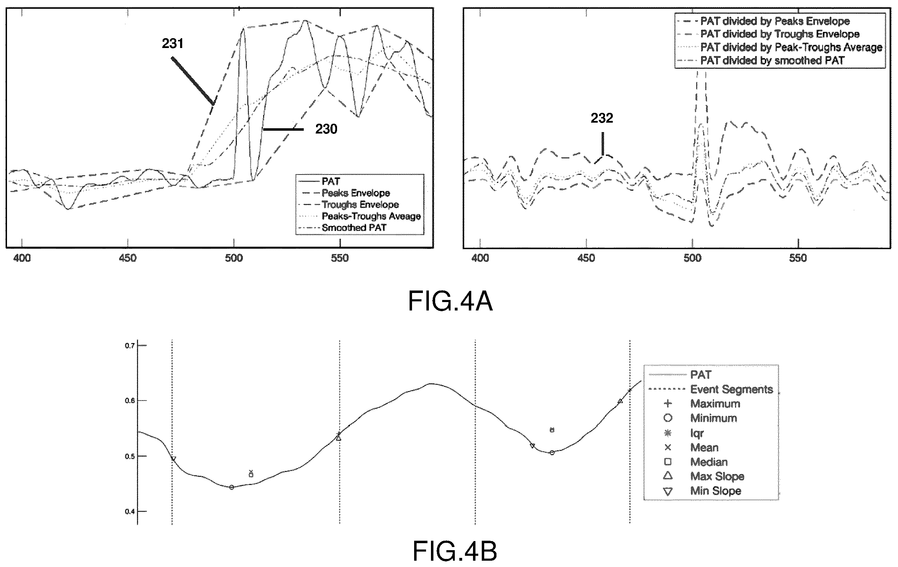

FIG.4A illustrates a detailed view of the effect of dividing each sample of the $PAT_{channel}$ signal 230 with the samples of the extracted peak envelope 231, throughs envelope, peaks-throughs average envelope, and a smoothed version of the $PAT_{channel}$ signal. The peak or troughs envelope corresponds to the local maxima and local minima amplitude values in the $PAT_{channel}$ signal, while the peak-troughs average envelope corresponds to the mean of the peak and troughs envelope. As it can be seen, the baseline-invariant signal 232 resulting from dividing the samples of the original $PAT_{channel}$ signal 230 with the upper or lower amplitude, the mean of the upper and lower amplitude, or the amplitude of the smoothed version of the signal substantially eliminates the amplitude depression caused by the VAR, while it maintains the integrity of the morphology and the relative amplitude variations of the respective event segments.

Once a baseline-invariant signal is calculated, the apparatus proceeds to characterize the event segments. To do so, the apparatus derives various features characterizing the event segments from the baseline-invariant version of the signal. The characterizing features comprise features such as for example: a maximum and/or a minimum amplitude of the event segment, a magnitude of the event segment; a median and/or a mean amplitude of the event segment; a quartile amplitude of the event segment; a duration of the vasoconstriction event; a duration of the amplitude drop period; a duration of the amplitude rise period of the vasoconstriction event; a steepness of the amplitude drop period; a steepness of the amplitude rise period of the vasoconstriction event; the full width at half maximum of the event segment, and so on.

To derive these features, the apparatus calculates in step 122 various characteristics points from the portion of the baseline-invariant signal corresponding to the event segment. The characteristic points are described in terms of amplitude and/or time. For example, characteristics points could be the sample of the event segment with the maximum amplitude value, the sample of the event segment with a minimum amplitude value, the first and last samples of the event segment, and so on.

The apparatus then uses the information obtained from the derived characteristic points to derive the various features characterising the event segment. To do so, in step 123, the apparatus relates the obtained information to derive the various features. For example, the magnitude of the event segment can be calculated as the difference between the maximum and the minimum amplitude values observed in the event segment. In other words, the magnitude of the amplitude drop of the event segment can be calculated as the difference between the amplitude value of the first sample or the sample with the maximum amplitude value and the amplitude value of the sample with the minimum amplitude value. Typically, the first sample coincides or substantially coincides with the sample with the maximum amplitude value. Herein, the first sample or the sample with maximum amplitude value acts as a reference point, while the sample with minimum amplitude value acts as a baseline point, and their respective amplitude values as a reference amplitude value and a baseline amplitude value. The duration of the event segment can be calculated as the difference in time between the first and the last samples of the event segment. The steepness of the amplitude drop of the event segment can be calculated as the ratio of the amplitude difference and the time difference of the first sample or the sample with the maximum amplitude value and the sample with the minimum amplitude value, and so on.

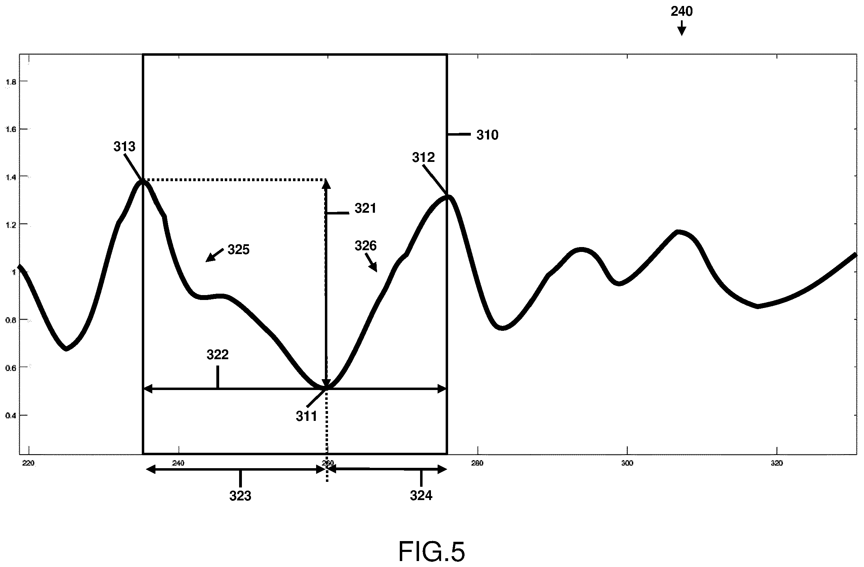

FIG.5 shows an example of event segment 310 of the baseline-invariant signal 240 illustrating various characteristic points allowing the calculation of various features characterizing the event segment. In this example, the reference amplitude value and the baseline amplitude values correspond to the signal's samples 313 and 311 with a maximum and minimum amplitude values, respectively. The duration 322 of the event segment is identified by the samples 313 and 312 corresponding to the first and the last samples in the event segment. The amplitude drop portion of the event segment and the amplitude rise portion of the event segment correspond to the slopes 325 and 326 with respective duration 323 and 324. In this example, the steepness of the amplitude drop is simplistically illustrated as the absolute magnitude 321 of the amplitude drop. Similarly, the steepness of the amplitude rise period is herein illustrated as the absolute magnitude between the samples 312 and 311.

Alternatively, the features characterising the event segment on an absolute scale can be derived by relating the amplitude of the signal portion corresponding to the event segment to a baseline level. For example, this can be done by dividing $PAT_{channel}$ signal portion corresponding to an event segment, by a baseline level for the event segment. For example, the baseline level for the event segment can be:

•    the maximum amplitude value of the event segment, i.e., its peak value;

- the minimum amplitude value of the event segment, i.e., its trough value; or
- the mean or median amplitude value of the event segment;
- the quartile or any percentile amplitude value of the event segment; or
- the amplitude value of any other characteristic point of the event segment, such as the point of maximum upward or downward slope of the event segment, or
- a smoothed version of the event segment.

As can be seen from this list, the baseline level for the event segments can be a baseline value or a baseline signal. FIG.4B illustrates an example of possible baseline values for two event segments which can be used to relate the amplitude of the event segment to a baseline level, e.g., by dividing the portion of the $PAT_{channel}$ signal by the baseline level corresponding to the event segment. In this figure, each event segment is indicated by a pair of dashed lines. The amplitude values indicating the maximum amplitude value, the minimum amplitude value, the mean amplitude value, the median amplitude value, as well as the lower quartile amplitude value, lqr, and the amplitude values at the maximum and the minimum slope of the event segments, are illustrated.

Dividing the portion of the $PAT_{channel}$ signal corresponding to the event segment by the baseline amplitude value for the event segment results in obtaining a baseline-invariant portion for the respective event segment. Thus, the output in step 121 is the baseline-invariant signal portion for the respective event segments. Once the baseline-invariant signal portions for the respective event segments are calculated, the apparatus proceeds to perform step 122 and step 123 as described above to derive the characteristics features for the respective events. That is, in step 122 the apparatus derives various characteristic points from the respective baseline-invariant signal portions corresponding to the respective event segments. Then, in step 123, the apparatus uses the information obtained from the characteristic points for the respective event segments to derive the various features characterising the respective event segments in the same manner as described above. For example, the magnitude of the event segment can be calculated as the difference between the maximum and the minimum amplitude values. The magnitude of the amplitude drop of the event segment can be calculated as the difference between the amplitude value of the first sample or the sample with the maximum amplitude value and the amplitude value of the sample with the minimum amplitude value, and so on.

Alternatively, any magnitude-related characteristic features such as features calculated using one or more amplitude values of the signal portion corresponding to the event segment can be obtained on a relative scale rather than on an absolute scale. In this case, step 121 is omitted and the apparatus derives the various characteristics features from the original, unmodified $PAT_{channel}$ signal 230 as shown in the third plot of FIG.3. In this case, to correct for the amplitude fluctuations caused by the VAR episode, the characteristics features affected by the VAR such as the magnitude and the steepness features, are calculated on a relative scale. To do so, the characteristic amplitude points for the event segment such as the peak amplitude, the trough amplitude, the peak-to-trough average amplitude, or the percentile value are derived as detailed above with reference to FIG.5. Differently from above, herein, the magnitude of the event segment is derived by, for example, calculating a relative difference between the reference and baseline amplitude values. Similarly, other amplitude-related features characterising the event segment which are derived based on one or more amplitude values derived from the signal portion corresponding to the event segment such as the steepness features can be derived on a relative scale.

The output of step 120 is, thus, one or more features characterizing the event segments by means of at least a magnitude measure characterizing the intensity of the event segment, and, possibly, one or more additional features such as a duration of the event segment, a duration of the amplitude drop period of the event segment; a duration of the amplitude rise period of the event segment, a steepness of the amplitude drop period of the event segment, a steepness of the amplitude rise period of the event segment, and a full width at half maximum of the event segment.

In the last step of the method, i.e., step 130, the method proceeds to detect sleep-disturbing events 14 based on the determined one or more features characterizing the vasoconstriction events. A sleep-disturbing event can be detected for example based on at least the magnitude measure of the vasoconstriction event. If the magnitude measure is above a predetermined threshold value, the vasoconstriction event is considered to be a sleep-disturbing event. In addition to the magnitude measure, other features characterizing the vasoconstriction event can be also taken into account. These additional features can further characterize the vasoconstriction event in terms of its morphological profile. A morphological profile may for example be linked to the shape of the vasoconstriction event. For example, at least one or a combination of the duration of the event segment, the duration of the amplitude drop period of the event segment; a duration of the amplitude rise period of the event segment, a steepness of the amplitude drop period of the event segment, a steepness of the amplitude rise period of the event segment, and a full width at half maximum of the event segment may be further taken into account. In such a case, a parameterized cost function can be evaluated which takes the considered features into account to evaluate whether a vasoconstriction event is a sleep-disturbing event.

This step of detecting sleep-disturbing events can be implemented by means of a classifier that has been developed to perform the step for detecting a sleep-disturbing event. The classifier thus implements the parameterized cost function by designing and inputting a set of rules for the detection. These rules can, as detailed above, be based on threshold values for the respective one or more features or for a respective combination of features that examine how well the features of an event comply with the criteria defined by the rules. The threshold values for the respective features can for example be derived based on measurements obtained during a clinical trial. From these measurements, first sleep-disturbing events are identified by means of manual or computer-aided scoring and then threshold values for the respective features are determined from the identified sleep-disturbing events. Further, the features can be ordered according to their accuracy in detecting a certain sleep-disturbing event, and then one or more most optimal features can be selected for the detection of a respective sleep-disturbing event. Using only the most optimal features for the detection of the sleep-disturbing events allows simplifying the classifier implementation without sacrificing detection accuracy. The thus developed classifier can then evaluate the parameterized cost function implementing these rules to determine whether an event is a sleep-disturbing event or not.

The apparatus 104 can further provide an indication of certain medical conditions and physiological states based upon the concurrence of sleep-disturbing events characterized with specific features. For example, by analyzing the concurrence of vasoconstriction events with certain features such as a certain minimum event duration and a certain minimum distance between the minimum and maximum sample value, i.e., the duration of the amplitude drop of the event, and/or features observed in other physiological signals, such as an increase in pulse rate or heart rate and/or a decrease in blood oxygen saturation and/or a limb movement during the event. The limb movement can be voluntary or involuntary movement and can for example be picked up by an accelerometer. This concurrence of the sleep-disturbing events and the additional physiological features can further aid the medical personnel in determining certain medical conditions and physiological states of the individual.

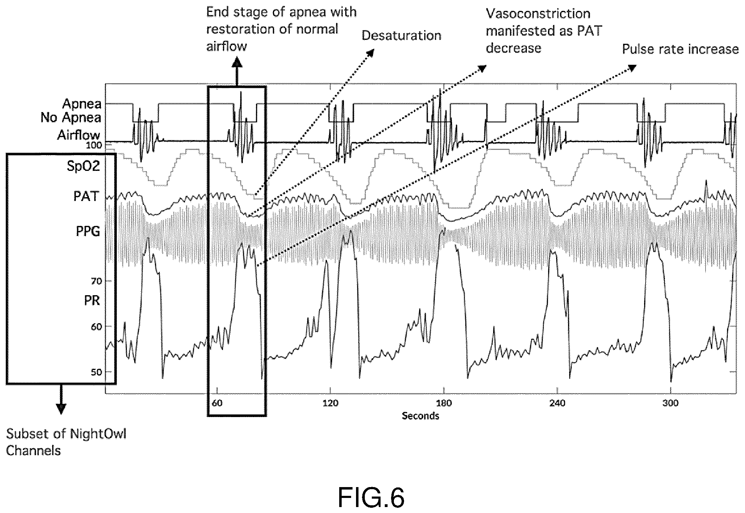

FIG.6 illustrates an example of a respiratory event, in this example an apnea event. As it can be seen from the figure, the end-stage of the apnea event coincides with a decrease in the $PAT_{channel}$ signal amplitude and/or an increase in pulse rate, PR, and/or a decrease in $SpO_2$.

As described above, the present solution can improve the accuracy of determining various characterizing features from the physiological signal indicative of the peripheral arterial tone and, thus, can improve the accuracy of detecting sleep-disturbing events from such a physiological signal. Further, as described above, the method can provide an indication of the concurrence of the sleep-disturbing events and, possibly, features observed in other physiological signals to provide additional information which can aid the medical personnel in determining certain medical conditions and physiological states of the individual.

Furthermore, although the above-presented observations and methodology are described with respect to optical plethysmography, the skilled person will recognize that the same observations and methodology can be readily applied to a physiological signal obtained by means of pneumatic plethysmography.

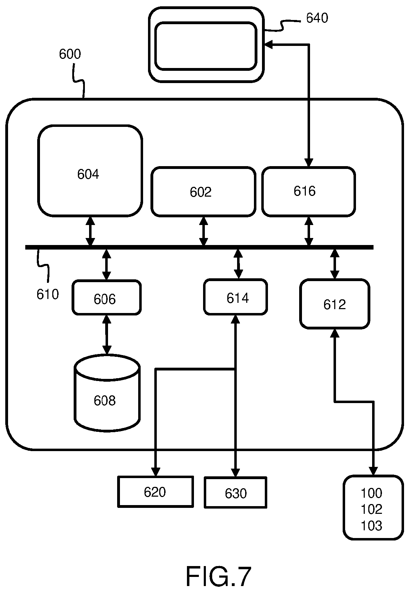

FIG.7 shows a suitable computing system 600 enabling to implement embodiments of the method for detecting sleep-disturbing events according to the invention. Computing system 600 may, in general, be formed as a suitable general-purpose computer and comprise a bus 610, a processor 602, a local memory 604, one or more optional input interfaces 614, one or more optional output interfaces 616, a communication interface 612, a storage element interface 606, and one or more storage elements 608. Bus 610 may comprise one or more conductors that permit communication among the components of the computing system 600. Processor 602 may include any type of conventional processor or microprocessor that interprets and executes programming instructions. Local memory 604 may include a random-access memory, RAM, or another type of dynamic storage device that stores information and instructions for execution by processor 602 and/or a read-only memory, ROM, or another type of static storage device that stores static information and instructions for use by processor 602. The processor may thus execute the instructions stored in the local memory to perform various steps of the method described above. Input interface 614 may comprise one or more conventional mechanisms that permit an operator or user to input information to the computing device 600, such as a keyboard 620, a mouse 630, a pen, voice recognition, etc. and/or one or more PPG sensors. Output interface 616 may comprise one or more conventional mechanisms that output information to the operator or user, such as a display 640, etc. Communication interface 612 may comprise any transceiver-like mechanism such as for example one or more Ethernet interfaces that enables computing system 600 to communicate with other devices and/or systems, for example with other computing devices 701, 702, 703. The processing of the signal obtained from the one or more PPG sensors may thus be processed remotely by the other computing devices. The communication interface 612 of computing system 600 may be connected to such another computing system by means of a local area network, LAN, or a wide area network, WAN, such as for example the internet. Storage element interface 606 may comprise a storage interface such as for example a Serial Advanced Technology Attachment, SATA, interface or a Small Computer System Interface, SCSI, for connecting bus 610 to one or more storage elements 608, such as one or more local disks, for example, SATA disk drives, and control the reading and writing of data to and/or from these storage elements 608. Although the storage element(s) 608 above is/are described as a local disk, in general, any other suitable computer-readable media such as a removable magnetic disk, optical storage media such as a CD or DVD, -ROM disk, solid state drives, flash memory cards, ... could be used. Computing system 600 could thus correspond to a circuitry for processing the signal obtained from one or more PPG sensors to detect, therefrom, sleep-disturbing events as described above with reference to FIG.1.

As used in this application, the term "circuitry" may refer to one or more or all of the following:

(a) hardware-only circuit implementations such as implementations in only analog and/or digital circuitry and
(b) combinations of hardware circuits and software, such as (as applicable):

(i) a combination of analog and/or digital hardware circuit(s) with software/firmware and
(ii) any portions of hardware processor(s) with software (including digital signal processor(s)), software, and memory(ies) that work together to cause an apparatus, such as a mobile phone or server, to perform various functions) and

(c) hardware circuit(s) and/or processor(s), such as microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example, and if applicable to the particular claim element, a baseband integrated circuit or processor integrated circuit for a mobile device or a similar integrated circuit in a server, a cellular network device, or other computing or network device.

It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A computer-implemented method for detecting sleep-disturbing events from a signal (230) indicative of a peripheral arterial tone of an individual, the signal being affected by a venoarteriolar reflex, the method comprises:

   - determining (110) a vasoconstriction event (310, 110_1 - 110_n) from changes in the signal (230, 240);
   - deriving (122) a reference amplitude value (313) and a baseline amplitude value (311) for the vasoconstriction event (310, 110_1 - 110_n), the reference amplitude value being different from the baseline amplitude value;
   - relating (123) the reference amplitude value (311) for the vasoconstriction event to the baseline amplitude value (313) for the vasoconstriction event, thereby obtaining a magnitude measure (321) for the vasoconstriction event; and
   - detecting (130), therefrom, a sleep-disturbing event.

2. The computer-implemented method according to claim 1, wherein deriving (122) comprises: calculating a baseline-invariant signal (232) for the vasoconstriction event, selecting, therefrom, the reference amplitude value (313) and the baseline amplitude value (311) for the vasoconstriction event, the reference and the baseline amplitude values corresponding to amplitude values for the vasoconstriction event allowing calculation of the magnitude measure for the vasoconstriction event.

3. The computer-implemented method according to claim 2, wherein the calculating comprises dividing a signal portion (310) corresponding to the vasoconstriction event with a baseline (231, 313) for the vasoconstriction event.

4. The computer-implemented method according to claims 2 or 3, wherein the relating (123) comprises calculating, from the reference amplitude value (311) and the baseline amplitude value (313), an absolute magnitude (321) for the vasoconstriction event.

5. The computer-implemented method according to any one of claims 2 to 4, wherein the baseline (231,313) for the vasoconstriction event is derived by calculating an envelope of the signal characterizing the venoarteriolar reflex in the signal such as a peak envelope (231), a troughs envelope, a peak-to-troughs average envelope, a percentile value-based envelope, or a smoothed version of the signal, or, by calculating an amplitude value for the vasoconstriction event characterizing the venoarteriolar reflex for the vasoconstriction event, such as a peak amplitude (311), a through amplitude, a peak-to-trough average amplitude, or a percentile value for the vasoconstriction event.

6. The computer-implemented method according to claim 1, wherein the reference amplitude value and the baseline amplitude value for the vasoconstriction event are respectively derived by calculating a peak amplitude, a through amplitude, a peak-to-trough average amplitude, or a percentile value for the vasoconstriction event.

7. The computer-implemented method according to claim 6, wherein the relating (123) comprises calculating, from the reference amplitude value and the baseline amplitude value for the vasoconstriction event, a relative magnitude for the vasoconstriction event.

8. The computer-implemented method according to any one of claims 1 to 7, wherein the detecting (130) comprises identifying a vasoconstriction event (310, 110_1 - 110_n) **characterized by** a magnitude measure (321) above a pre-determined value.

9. The computer-implemented method according to claim 8, wherein the identifying further takes into account at least one of a duration (322) of the vasoconstriction event, a duration (323) of an amplitude drop period and/or a duration (324) amplitude rise period of the vasoconstriction event, a steepness (325) of the amplitude drop period and/or a steepness (326) of the amplitude rise period of the vasoconstriction event.

10. The computer-implemented method according to any one of claims 1 to 9, wherein the determining (110) comprises identifying a portion of the signal (230, 240) **characterized by** an amplitude drop (311) followed by an amplitude increase (312).

11. The computer-implemented method according to claim 10, wherein the determining (110) further takes into account at least one of a duration (322) of a period of the amplitude drop and a period of the amplitude increase of the portion of the signal, a duration (323) of a period of the amplitude drop and/or a duration (324) a period of the amplitude increase of the portion of the signal, a steepness (325) of a period of the amplitude drop and/or a steepness (326) of a period of the amplitude increase of the portion of the signal.

12. An apparatus configured to detect sleep-disturbing events from a signal (230) indicative of a peripheral arterial tone of an individual affected by a venoarteriolar reflex, the apparatus comprising means configured to perform:

   - determining (110) a vasoconstriction event (310, 110_1 - 110_n) from changes in the signal (230,240);
   - deriving (122) a reference amplitude value (313) and a baseline amplitude value (311) for the vasoconstriction event (310, 110_1 - 110_n), the reference amplitude value being different from the baseline amplitude value;
   - relating (123) the reference amplitude value (311) for the vasoconstriction event to the baseline amplitude value (313) for the vasoconstriction event, thereby obtaining a magnitude measure (321) for the vasoconstriction event; and
   - detecting (130), therefrom, a sleep-disturbing event.

13. A computer program product comprising computer-executable instructions for causing a computer to perform the computer-implemented method according to any one of claims 1 to 11.

14. A computer readable storage medium comprising computer-executable instructions for performing the computer-implemented method according to any one of claims 1 to 11 when the computer-implemented method is run as a program on a computer.

15. The computer readable storage medium according to claim 14, wherein the computer-implemented method further comprises obtaining a signal indicative of changes in pulsatile blood volume, obtained by means of plethysmography at a selected anatomical location of the individual, and, deriving, therefrom, the signal (230) indicative of changes in peripheral arterial tone.

**Patentansprüche**

1. Computer-implementiertes Verfahren zum Erfassen von schlafstörenden Ereignissen aus einem Signal (230), das einen peripheren arteriellen Tonus eines Individuums angibt, wobei das Signal durch einen venoarteriellen Reflex beeinflusst wird, wobei das Verfahren umfasst:

   - Bestimmen (110) eines vasokonstriktorischen Ereignisses (310, 110_1 - 110_n) aus Veränderungen in dem Signal (230, 240);
   - Ableiten (122) eines Referenz-Amplitudenwerts (313) und eines Basislinien-Amplitudenwerts (311) für das vasokonstriktorische Ereignis (310, 110_1 - 110_n), wobei sich der Referenz-Amplitudenwert von dem Basislinien-Amplitudenwert unterscheidet;
   - Korrelieren (123) des Referenz-Amplitudenwerts (311) für das vasokonstriktorische Ereignis mit dem Basislinien-Amplitudenwert (313) für das vasokonstriktorische Ereignis, wodurch ein Maß der Größe (321) für das vasokonstriktorische Ereignis erhalten wird; und
   - Erfassen (130) eines schlafstörenden Ereignisses daraus.

2. Computer-implementiertes Verfahren nach Anspruch 1, wobei das Ableiten (122) umfasst: Berechnen eines basislinien-invarianten Signals (232) für das vasokonstriktorische Ereignis, Auswählen des Referenz-Amplitudenwerts (313) und des Basislinien-Amplitudenwerts (311) für das vasokonstriktorische Ereignis daraus, wobei die Referenz- und die Basislinien-Amplitudenwerte Amplitudenwerten für das vasokonstriktorische Ereignis entsprechen, welche die Berechnung des Maßes der Größe für das vasokonstriktorische Ereignis ermöglichen.

3. Computer-implementiertes Verfahren nach Anspruch 2, wobei das Berechnen das Dividieren eines Signalabschnitts (310) entsprechend dem vasokonstriktorischen Ereignis durch eine Basislinie (231, 313) für das vasokonstriktorische Ereignis umfasst.

4. Computer-implementiertes Verfahren nach den Ansprüchen 2 oder 3, wobei das Korrelieren (123) das Berechnen einer absoluten Größenordnung (321) für das vasokonstriktorische Ereignis aus dem Referenz-Amplitudenwert (311) und dem Basislinien-Amplitudenwert (313) umfasst.

5. Computer-implementiertes Verfahren nach einem der Ansprüche 2 bis 4, wobei die Basislinie (231, 313) für das vasokonstriktorische Ereignis durch Berechnen einer Hüllkurve des Signals abgeleitet wird, die den venoarteriellen Reflex in dem Signal kennzeichnet, wie etwa eine Spitzenhüllkurve (231), eine Tiefstwert-Hüllkurve, eine Durchschnittshüllkurve von Spitzenwert zu Tiefstwert, eine Hüllkurve auf der Grundlage von Perzentilwerten oder eine geglättete Version des Signals, oder durch Berechnen eines Amplitudenwerts für das vasokonstriktorische Ereignis, der den venoarteriellen Reflex für das vasokonstriktorische Ereignis kennzeichnet, wie etwa eines Spitzen-Amplitudenwerts (311), eines Tiefst-Amplitudenwerts, eines Durchschnitts-Amplitudenwerts von Spitzenwert zu Tiefstwert oder eines Perzentilwerts für das vasokonstriktorische Ereignis.

6. Computer-implementiertes Verfahren nach Anspruch 1, wobei der Referenz-Amplitudenwert und der Basislinien-Amplitudenwert für das vasokonstriktorische Ereignis jeweils durch Berechnen einer Spitzenwert-Amplitude, einer Tiefstwert-Amplitude, einer Durchschnittsamplitude von Spitzenwert zu Tiefstwert, oder eines Perzentilwerts für das vasokonstriktorische Ereignis abgeleitet werden.

7. Computer-implementiertes Verfahren nach Anspruch 6, wobei das Korrelieren (123) das Berechnen einer relativen Größenordnung für das vasokonstriktorische Ereignis aus dem Referenz-Amplitudenwert und dem Basislinien-Amplitudenwert für das vasokonstriktorische Ereignis umfasst.

8. Computer-implementiertes Verfahren nach einem der Ansprüche 1 bis 7, wobei das Erfassen (130) das Identifizieren eines vasokonstriktorischen Ereignisses (310, 110_1 - 110_n) umfasst, das durch ein Maß der Größe (321) über einem vorbestimmten Wert gekennzeichnet ist.

9. Computer-implementiertes Verfahren nach Anspruch 8, wobei das Identifizieren ferner zumindest eine von einer Dauer (322) des vasokonstriktorischen Ereignisses, einer Dauer (323) einer abfallenden Amplitudenperiode und/oder einer Dauer (324) einer ansteigenden Amplitudenperiode des vasokonstriktorischen Ereignisses, einer Steilheit (325) der abfallenden Amplitudenperiode und/oder einer Steilheit (326) der ansteigenden Amplitudenperiode des vasokonstriktorischen Ereignisses berücksichtigt.

10. Computer-implementiertes Verfahren nach einem der Ansprüche 1 bis 9, wobei das Bestimmen (110) das Identifizieren eines Abschnitts des Signals (230, 240) umfasst, das durch einen Abfall der Amplitude (311) gefolgt von einem Anstieg der Amplitude (312) gekennzeichnet ist.

11. Computer-implementiertes Verfahren nach Anspruch 10, wobei das Bestimmen (110) ferner zumindest eine von einer Dauer (322) einer Periode des Amplitudenabfalls und einer Periode des Amplitudenanstiegs des Abschnitts des Signals, einer Dauer (323) einer Periode des Amplitudenabfalls und/oder einer Dauer (324) einer Periode des Amplitudenanstiegs des Abschnitts des Signals, einer Steilheit (325) einer Periode des Amplitudenabfalls und/oder einer Steilheit (326) einer Periode des Amplitudenanstiegs des Abschnitts des Signals berücksichtigt.

12. Vorrichtung, die dazu ausgestaltet ist, schlafstörende Ereignisse aus einem Signal (230) zu erfassen, das einen peripheren arteriellen Tonus eines Individuums angibt, wobei das Signal durch einen venoarteriellen Reflex beeinflusst wird, wobei die Vorrichtung Mittel umfasst zum:

- Bestimmen (110) eines vasokonstriktorischen Ereignisses (310, 110_1 - 110_n) aus Veränderungen in dem Signal (230, 240);
- Ableiten (122) eines Referenz-Amplitudenwerts (313) und eines Basislinien-Amplitudenwerts (311) für das vasokonstriktorische Ereignis (310, 110_1 - 110_n), wobei sich der Referenz-Amplitudenwert von dem Basislinien-Amplitudenwert unterscheidet;
- Korrelieren (123) des Referenz-Amplitudenwerts (311) für das vasokonstriktorische Ereignis mit dem Basislinien-Amplitudenwert (313) für das vasokonstriktorische Ereignis, wodurch ein Maß der Größe (321) für das vasokonstriktorische Ereignis erhalten wird; und
- Erfassen (130) eines schlafstörenden Ereignisses daraus.

13. Computerprogrammprodukt, umfassend computerausführbare Anweisungen, um einen Computer zu veranlassen, das computer-implementierte Verfahren nach einem der Ansprüche 1 bis 11 auszuführen.

14. Computerlesbares Speichermedium, umfassend computerausführbare Anweisungen zum Durchführen des computer-implementierten Verfahrens nach einem der Ansprüche 1 bis 11, wenn das computer-implementierte Verfahren als Programm auf einem Computer ausgeführt wird.

15. Computerlesbares Speichermedium nach Anspruch 14, wobei das computer-implementierte Verfahren ferner das Erhalten eines Signals, das Veränderungen im pulsatilen Blutvolumen angibt, die mittels Plethysmographie an einer ausgewählten anatomischen Position des Individuums erhalten werden, und das Ableiten des Signals (230), das Veränderungen im peripheren arteriellen Tonus angibt, aus diesem umfasst.

## Revendications

1. Procédé mis en œuvre par ordinateur pour détecter des événements perturbant le sommeil à partir d'un signal (230) indicatif d'un tonus artériel périphérique d'un individu, le signal étant affecté par un réflexe veino-artériolaire, le procédé comprend :

- la détermination (110) d'un événement de vasoconstriction (310, 110_1 - 110_n) à partir des changements du signal (230, 240) ;
- la dérivation (122) d'une valeur d'amplitude de référence (313) et d'une valeur d'amplitude de ligne de base (311) pour l'événement de vasoconstriction (310, 110_1 - 110_n), la valeur d'amplitude de référence étant différente de la valeur d'amplitude de ligne de base ;
- la mise en relation (123) de la valeur d'amplitude de référence (311) pour l'événement de vasoconstriction avec la valeur d'amplitude de ligne de base (313) pour l'événement de vasoconstriction, obtenant ainsi une mesure de grandeur (321) pour l'événement de vasoconstriction ; et
- la détection (130), à partir de celle-ci, d'un événement perturbant le sommeil.

2. Procédé mis en œuvre par ordinateur selon la revendication 1, dans lequel la dérivation (122) comprend : le calcul d'un signal invariant par rapport à la ligne de base (232) pour l'événement de vasoconstriction, la sélection, à partir de celui-ci, de la valeur d'amplitude de référence (313) et de la valeur d'amplitude de ligne de base (311) pour l'événement de vasoconstriction, les valeurs d'amplitude de référence et de ligne de base correspondant à des valeurs d'amplitude pour l'événement de vasoconstriction permettant le calcul de la mesure de grandeur pour l'événement de vasoconstriction.

**3.** Procédé mis en œuvre par ordinateur selon la revendication 2, dans lequel le calcul comprend la division d'une partie de signal (310) correspondant à l'événement de vasoconstriction avec une ligne de base (231, 313) pour l'événement de vasoconstriction.

**4.** Procédé mis en œuvre par ordinateur selon la revendication 2 ou 3, dans lequel la mise en relation (123) comprend le calcul, à partir de la valeur d'amplitude de référence (311) et de la valeur d'amplitude de ligne de base (313), d'une grandeur absolue (321) pour l'événement de vasoconstriction.

**5.** Procédé mis en œuvre par ordinateur selon l'une quelconque des revendications 2 à 4, dans lequel la ligne de base (231, 313) pour l'événement de vasoconstriction est dérivée en calculant une enveloppe du signal caractérisant le réflexe veino-artériolaire dans le signal telle qu'une enveloppe de crête (231), une enveloppe de creux, une enveloppe moyenne crête-à-creux, une enveloppe basée sur une valeur de centile, ou une version lissée du signal, ou, en calculant une valeur d'amplitude pour l'événement de vasoconstriction caractérisant le réflexe veino-artériolaire pour l'événement de vasoconstriction, telle qu'une amplitude de crête (311), une amplitude de creux, une amplitude moyenne crête-à-creux, ou une valeur de centile pour l'événement de vasoconstriction.

**6.** Procédé mis en œuvre par ordinateur selon la revendication 1, dans lequel la valeur d'amplitude de référence et la valeur d'amplitude de ligne de base pour l'événement de vasoconstriction sont respectivement dérivées en calculant une amplitude de crête, une amplitude de creux, une amplitude moyenne crête-à-creux ou une valeur de centile pour l'événement de vasoconstriction.

**7.** Procédé mis en œuvre par ordinateur selon la revendication 6, dans lequel la mise en relation (123) comprend le calcul, à partir de la valeur d'amplitude de référence et de la valeur d'amplitude de ligne de base pour l'événement de vasoconstriction, d'une grandeur relative pour l'événement de vasoconstriction.

**8.** Procédé mis en œuvre par ordinateur selon l'une quelconque des revendications 1 à 7, dans lequel la détection (130) comprend l'identification d'un événement de vasoconstriction (310, 110_1 - 110_n) **caractérisé par** une mesure de grandeur (321) supérieure à une valeur prédéterminée.

**9.** Procédé mis en œuvre par ordinateur selon la revendication 8, dans lequel l'identification prend en compte en outre au moins l'une parmi une durée (322) de l'événement de vasoconstriction, une durée (323) d'une période de chute d'amplitude et/ou une durée (324) d'une période d'élévation d'amplitude de l'événement de vasoconstriction, une pente (325) de la période de chute d'amplitude et/ou une pente (326) de la période d'élévation d'amplitude de l'événement de vasoconstriction.

**10.** Procédé mis en œuvre par ordinateur selon l'une quelconque des revendications 1 à 9, dans lequel la détermination (110) comprend l'identification d'une partie du signal (230, 240) **caractérisée par** une chute d'amplitude (311) suivie d'une augmentation d'amplitude (312).

**11.** Procédé mis en œuvre par ordinateur selon la revendication 10, dans lequel la détermination (110) prend en compte en outre au moins l'une parmi une durée (322) d'une période de la chute d'amplitude et d'une période de l'augmentation d'amplitude de la partie du signal, une durée (323) d'une période de la chute d'amplitude et/ou une durée (324) d'une période de l'augmentation d'amplitude de la partie du signal, une pente (325) d'une période de la chute d'amplitude et/ou une pente (326) d'une période de l'augmentation d'amplitude de la partie du signal.

**12.** Appareil configuré pour détecter des événements perturbant le sommeil à partir d'un signal (230) indicatif d'un tonus artériel périphérique d'un individu affecté par un réflexe veino-artériolaire, l'appareil comprenant des moyens configurés pour réaliser :

- la détermination (110) d'un événement de vasoconstriction (310, 110_1 - 110_n) à partir des changements du signal (230, 240) ;
- la dérivation (122) d'une valeur d'amplitude de référence (313) et d'une valeur d'amplitude de ligne de base (311) pour l'événement de vasoconstriction (310, 110_1 - 110_n), la valeur d'amplitude de référence étant différente de la valeur d'amplitude de ligne de base ;
- la mise en relation (123) de la valeur d'amplitude de référence (311) pour l'événement de vasoconstriction avec la valeur d'amplitude de ligne de base (313) pour l'événement de vasoconstriction, obtenant ainsi une mesure de grandeur (321) pour l'événement de vasoconstriction ; et
- la détection (130), à partir de celle-ci, d'un événement perturbant le sommeil.

13. Produit de programme informatique comprenant des instructions exécutables par ordinateur pour amener un ordinateur à réaliser le procédé mis en œuvre par ordinateur selon l'une quelconque des revendications 1 à 11.

14. Support de stockage lisible par ordinateur comprenant des instructions exécutables par ordinateur pour réaliser le procédé mis en œuvre par ordinateur selon l'une quelconque des revendications 1 à 11 lorsque le procédé mis en œuvre par ordinateur est exécuté en tant que programme sur un ordinateur.

15. Support de stockage lisible par ordinateur selon la revendication 14, dans lequel le procédé mis en œuvre par ordinateur comprend en outre l'obtention d'un signal indicatif de changements de volume sanguin pulsatile, obtenu au moyen d'une pléthysmographie à un emplacement anatomique sélectionné de l'individu, et la dérivation, à partir de celui-ci, du signal (230) indicatif de changements du tonus artériel périphérique.

EP 4 208 085 B1

100
↓

```
┌─────────┐   12   ┌───────────────┐   14
│         │────────▶│               │────────▶
│   102   │         │      104      │
│         │         │               │
└─────────┘         └───────────────┘
```

FIG.1A

```
┌─────────────────────────────┐
│   DERIVE PAT SIGNAL FROM LIGHT │──── 111
│         INTENSITIES            │
└─────────────────────────────┘
              │
              ▼
┌─────────────────────────────┐
│   DETERMINE VASOCONSTRICTION  │──── 112
│   EVENT(S) IN THE PAT SIGNAL  │
└─────────────────────────────┘
              │
              ▼
┌ ─ ─ ─ ─ ─ ─ ─ ─ ─ ─ ─ ─ ─ ─ ┐
    CALCULATE BASELINE-INVARIANT   ──── 121
│            SIGNAL             │
└ ─ ─ ─ ─ ─ ─ ─ ─ ─ ─ ─ ─ ─ ─ ┘
              │
              ▼
┌─────────────────────────────┐
│   DERIVE CHARACTERISTIC POINTS,│──── 122
│   SUCH AS BASELINE AND REFERENCE │
│   AMPLITUDES, FOR THE EVENT(S) │
└─────────────────────────────┘
              │
              ▼
┌─────────────────────────────┐
│   RELATE CHARACTERISTIC POINTS │──── 123
│   SUCH AS REFERENCE AMPLITUDE TO │
│     BASELINE AMPLITUDE         │
└─────────────────────────────┘
              │
              ▼
┌─────────────────────────────┐
│    DETECT SLEEP DISTURBING    │──── 130
│          EVENT(S)             │
└─────────────────────────────┘
```

120

FIG.1B

FIG.2

FIG.3

EP 4 208 085 B1

FIG.4A

FIG.4B

FIG.5

EP 4 208 085 B1

FIG.6

FIG.7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7374540 B2

### Non-patent literature cited in the description

- **HAMUNEN et al.** Effect of pain on autonomic nervous system indices derived from photoplethysmography in healthy volunteers. *British Journal of Anesthesia*, 01 May 2012, vol. 108 (5), 838-844
- **BAR**. *An Illustrated Atlas of PAT Signals in Sleep Medicine*
- **CHATTERJEE et al.** Monte Carlo Analysis of Optical Interactions in Reflectance and Transmittance Finger Photoplethysmography. Sensors, 15 February 2019, vol. 19, 789